# EUROPEAN PATENT APPLICATION

(11) **EP 1 811 018 A1**
(43) Date of publication of application: **25.07.2007**
(21) Application number: 05793194.1
(22) Date of filing: 12.10.2005
(51) Int. Cl.: C12N 5/02, A61P 25/00, A61K 45/00, A61K 31/7088, A61K 35/28

(54) **BRAIN DISPOSITION MARROW PROGENITOR**

(30) Priority: 12.10.2004 JP 2004298170
(71) Applicant: TISSUE TARGETING JAPAN INC., Nagoya-shi, Aichi 458-0039 (JP)
(72) Inventor: SAWADA, Makoto, Kasugai-shi, Aichi 4870015 (JP); ONO, Kenji, Nagoya-shi, Aichi 4670053 (JP)
(74) Representative: Grünecker, Kinkeldey, Stockmair & Schwanhäusser Anwaltssozietät
(86) International application number: PCT/JP2005/018785
(87) International publication number: WO 2006/041088

(57) **Abstract**

The present inventors discovered that brain-localizing cells exist in the progenitor cell fraction of bone marrow cells. They also elucidated various characteristics of the brain-localizing cells. When these cells are infused into the bloodstream, they circulate in the blood and directly translocate into the cerebral parenchyma from the bloodstream. Furthermore, the present inventors succeeded in developing methods for efficiently preparing brain-localizing cells from bone marrow or bone marrow cells. These methods can be applied to less-invasive regenerative medicines targeting the brain and using autologous cells.

## Description

### Technical Field

The present invention relates to cells with brain-localizing activity, and methods for preparing the same.

### Background Art

The transport of substances and cells to the brain, which is the center of higher functions, is restricted by a barrier structure called blood-brain barrier. Therefore, it has been difficult to effectively treat the brain, except by surgical operation. Even when white blood cells such as monocytes, which circulate through the bloodstream, are collected and transplanted to adult animals, it is known that the cells do not transfer to the cerebral parenchyma, except when the blood-brain barrier is disrupted due to external factors (see Non-patent Document 1). On the other hand, microglia are present in the brain as macrophage-like cells; however, when microglia isolated from a neonatal brain are injected into blood, they are known to show brain-specific infiltration (see Non-patent Document 2). However, microglia are cells in the cerebral parenchyma, and to date, brain culture is the only method for obtaining large numbers of microglia.

Recent studies have shown that when bone marrow cells are collected from bone marrow, which serves as a reservoir for adult hematopoiesis, and then transplanted, some of the donor-derived cells translocate into the cerebral parenchyma (see Non-patent Documents 3 to 7). In most of these reports, host bone marrow cells were first removed by radiation exposure, and then the bone marrow transplantation was performed. The donor bone marrow stem cells settled in the host bone marrow a few months after transplant, and many donor-derived cells were also found in the cerebral parenchyma. Some of these reports report that some of the bone marrow cells that translocated into the brain then transdifferentiated into cells such as nerve cells and glial cells, which have different origins (see Non-patent Documents 4 to 6). It is also known that when cultured, some of the mesenchymal cells comprised in bone marrow cells can transdifferentiate into neural cells, liver cells and such (see Non-patent Documents 8 and 9). However, it has also been reported that the blood brain barrier is disrupted by radiation exposure (see Non-patent Document 10), and it is unclear what fraction of cells in the bone marrow translocate into the cerebral parenchyma, and at what stage after transplantation the brain-localizing cells translocate into the cerebral parenchyma.

[Non-patent Document 1] Imai, F., Sawada, M., Suzuki, H. et al. Neurosci Lett 237 1 pp.49-52 (1997)

[Non-patent Document 2] Sawada, M., Imai, F., Suzuki, H., et al. FEBS Lett 433 1-2 pp.37-40 (1998)

[Non-patent Document 3] Ono K, Takii T, Onozaki K, et al. Biochem Biophys Res Commun 262 3 pp.610-4 (1999)

[Non-patent Document 4] Mezey, E., Chandross, K. J., Harta, G, et al. Science 290 5497 pp.1779-82 (2000)

[Non-patent Document 5] Brazelton, T. R., Rossi, F. M., Keshet, G. I. et al. Science 290 5497 1775-9 (2000)

[Non-patent Document 6] Jiang, Y, Jahagirdar, B. N., Reinhardt, R. L. et al. Nature 418 6893 pp.41-9 (2002)

[Non-patent Document 7] Nakano, K., Migita, M., Mochizuki, H. et al. Transplantation 71 12 pp.1735-40 (2001)

[Non-patent Document 8] Woodbury, D., Schwarz, E. J., Prockop, D. J. et al. J Neurosci Res 61 4 pp.364-70 (2000)

[Non-patent Document 9] Schwartz, R. E., Reyes, M., Koodie, L. et al. J Clin Invest 109 10 pp.1291-302 (2002)

[Non-patent Document 10] Monje, M. L., Mizumatsu, S., Fike, J. R. et al. Nat Med 8 9 pp.955-62 (2002)

### Disclosure of the Invention

### [Problems to be Solved by the Invention]

The present invention was achieved in view of the above circumstances. An objective of the present invention is to identify cells with brain-localizing activity and to develop methods that enable the efficient acquisition of these cells. More specifically, an objective of the present invention is to provide brain-localizing cells, and methods for preparing the same.

### [Means to Solve the Problems]

The present inventors conducted dedicated studies to solve the above-mentioned problems, and discovered that brain-localizing (brain-oriented) cells are present in the progenitor cell fraction of mouse bone marrow cells. When these cells were transplanted under mild conditions that do not disrupt the blood brain barrier, they translocated into the cerebral parenchyma from the early stages after transplantation. Therefore, these cells were considered to translocate directly to the brain without going *via* host bone marrow, unlike cells derived from the transplanted cells, which are confirmed to appear in other organs one to two weeks after transplantation.

When bone marrow is transplanted, donor-derived bone marrow cells usually settle in the recipient's bone marrow, and then hematopoiesis by this bone marrow provides hemocytes to various peripheral tissues. Therefore, it is difficult to send out cells in a tissue-specific manner, and there was a need to at least consider the impact of going *via* bone marrow. When using the bone-marrow-derived brain-localizing cells of the present invention, the cells are infused into the bloodstream and then translocate directly into the cerebral parenchyma from the bloodstream through blood circulation; therefore, there is no need to consider the impact of going *via* bone marrow.

Although many of the cells observed in the brain after bone marrow transplantation grow in the brain, the cells of the present invention arrest in their G0/G1 phase in the cerebral parenchyma and exist in a growth-arrested state. The cells maintained relatively undifferentiated properties, and did not transdifferentiate into neural cells. The cells were confirmed to be stable in the cerebral parenchyma for several months, but the number of cells decreased with time. For example, GFP-expressing cells were also found in mouse brains 18 weeks after transplantation, but the number of cells was considerably less than in mouse brains up to four weeks after transplantation. Thus, the cells that translocated to the brain are considered to exist transiently in the brain because they are not stem cells. Accordingly, expression level, dose and such, which are of concern when conducting drug therapy or gene therapy, can be controlled relatively easily.

The present inventors examined means for separating and identifying these cells, and discovered effective means. These techniques discovered by the present inventors may be applied to less invasive brain-targeted regenerative medicines that use autologous cells.

The present inventors discovered cells with brain-localizing activity (brain-localizing bone marrow progenitor cells) from among the cells obtainable from bone marrow, as described above, and then conducted dedicated studies to succeed in elucidating the characteristics of these cells. The present inventors utilized these characteristics to successfully develop methods for efficiently preparing brain-localizing cells from bone marrow, and completed the present invention.

That is, the present invention relates to brain-localizing cells, and specifically to brain-localizing bone marrow progenitor cells obtained from bone marrow, and to methods for preparing the same. More specifically, the present invention provides:
[1] a bone marrow progenitor cell with brain-localizing activity, wherein the cell is an undifferentiated cell;
[2] the cell of [1], which is (a) ER-MP12-positive and/or (b) Lin-negative;
[3] the cell of [2], which is also (c) EpoR-positive;
[4] the cell of any of [1] to [3], which translocates to the brain without going through a recipient's bone marrow when administered to the recipient;
[5] the cell of any of [1] to [3], which exists in a growth-arrested state at G0/G1 phase in the cerebral parenchyma;
[6] the cell of any of [1] to [3], which does not transdifferentiate into a neural cell in the cerebral parenchyma;
[7] the cell of any of [1] to [3], which has the ability to pass through the blood brain barrier;
[8] a bone marrow progenitor cell fraction that substantially comprises cells with brain-localizing activity, wherein the fraction is prepared from bone marrow or bone marrow cells;
[9] the cell fraction of [8], wherein the cell component is a cell that is ER-MP 12-positive and/or Lin-negative;
[10] the cell fraction of [9], wherein the cell component is a cell that is also EpoR-positive;
[11] the cell fraction of any of [8] to [10], wherein the cell component is a cell that translocates to the brain without going through a recipient's bone marrow when the cell is administered to the recipient;
[12] the cell fraction of any of [8] to [10], wherein the cell component is a cell that exists in a growth-arrested state at G0/G1 phase in the cerebral parenchyma;
[13] the cell fraction of any of [8] to [10], wherein the cell component is a cell that does not transdifferentiate into a neural cell in the cerebral parenchyma;
[14] the cell fraction of any of [8] to [10], wherein the cell component is a cell with the ability to pass through the blood brain barrier;
[15] a method for obtaining a brain-localizing cell, which comprises the step of separating a bone marrow progenitor cell or a bone marrow progenitor cell fraction from bone marrow or bone marrow cells;
[16] the method of [15], which comprises the step of separating an undifferentiated cell or a cell fraction that substantially comprises said cell;
[17] the method of [16], wherein the undifferentiated cell is ER-MP12-positive and/or Lin-negative;
[18] the method of [17], wherein an ER-MP12-positive cell is separated using an anti-ER-MP 12 antibody, and/or an Lin-negative cell is separated using a lineage cocktail;
[19] the method of any of [16] to [18], which further comprises the step of separating an EpoR-positive cell, or a cell fraction substantially comprising said cell;
[20] the method of [19], wherein an EpoR-positive cell is separated using an anti-EpoR antibody;
[21] the method of any of [15] to [20], which further comprises the step of culturing cells with the addition of a WEHI-3B cell culture supernatant;
[22] a carrier for delivery to the brain, which comprises the bone marrow progenitor cell of any of [1] to [7], the cell fraction of any of [8] to [14], or the brain-localizing cell obtained by any of the methods of [15] to [21];
[23] a brain-localizing pharmaceutical agent, which comprises a biologically active substance that is comprised in the carrier of [22] for delivery into the brain;
[24] the agent of [23], wherein the biologically active substance has a therapeutic effect on a brain disease;
[25] a method for producing a brain-localizing cell comprising a biologically active substance, wherein the method comprises the steps of:
   (a) obtaining a brain-localizing cell from the bone marrow or bone marrow cells by the method of any of [15] to [21]; and
   (b) introducing a biologically active substance into the brain-localizing cell of step (a);
[26] the production method of [25], wherein the biologically active substance has a therapeutic effect on a brain disease;
[27] the production method of [25] or [26], wherein the biologically active substance is a nucleic acid;
[28] a kit for preparing a brain-localizing cell, which comprises at least two or more components selected from the following (a) to (d):
   (a) an anti-ER-MP12 antibody;
   (b) a Lineage cocktail;
   (c) an anti-EpoR antibody; and
   (d) a medium for culturing a bone marrow cell; and
[29] a kit for producing a brain-localizing cell, which comprises a biologically active substance, and the following (a) and (b) as components:
   (a) a bone marrow progenitor cell of any one of [1] to [7], or a cell fraction of any one of [8] to [14]; and
   (b) a medium for culturing a bone marrow progenitor cell of any one of [1] to [7].

The present invention further relates to:
[30] a bone marrow progenitor cell with brain-localizing activity, which is prepared by a method comprising the steps of:
   (a) separating a bone marrow progenitor cell or bone marrow progenitor cell fraction from bone marrow or bone marrow cells;
   (b) separating an undifferentiated cell or a cell fraction substantially comprising the cell;
   (c) separating an EpoR-positive and/or CD13-positive cell, or a cell fraction substantially comprising the cell; and
   (d) culturing the cell by adding a WEHI-3B cell culture supernatant;
[31] a method for treating a brain disease, which comprises the step of administering any of the above-mentioned bone marrow progenitor cells, carriers for transfer into the brain, and brain-localizing pharmaceutical agents to an individual (such as a patient);
[32] use of any of the above-mentioned bone marrow progenitor cells and carriers for transfer into the brain for producing a brain-localizing pharmaceutical agent (therapeutic agent for a brain disease); and
[33] a composition comprising any of the above-mentioned bone marrow progenitor cells, carriers for transfer into the brain, and brain-localizing pharmaceutical agents together with a pharmaceutically acceptable carrier.

### Brief Description of the Drawings

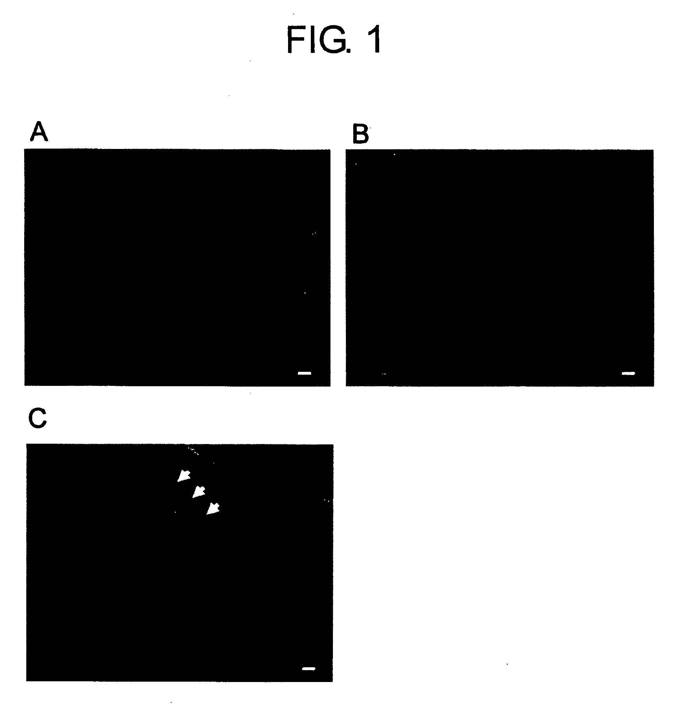

Fig. 1 shows photographs indicating the leakage of Evans blue dye from blood vessels at the mouse blood brain barrier. The fluorescent images show the cerebral cortices of (A) a normal B6 mouse, (B) a 5-FU-treated B6 mouse, and (C) a B6 mouse physically injured using an injection needle. Dye leakage is not seen in mice A and B, but mouse C shows significant dye leakage. The arrows indicate needle scars. The scale bars indicate 40 µm.

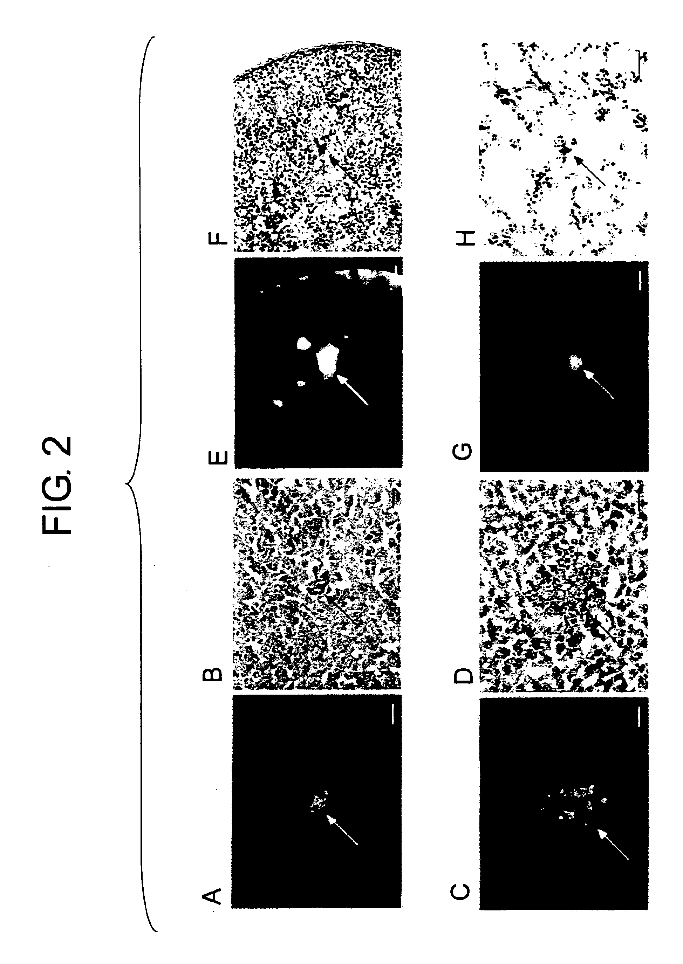

Fig. 2 shows photographs of GFP-expressing cells in mouse tissues on day 7 after bone marrow transplantation. The fluorescent images show the brain, liver, spleen, and lung (A, C, E, and G) of mice on day 7 after transplantation, and hematoxylin-eosin-stained images of the same sections (B, D, F, and H). The arrows indicate the same cells. The scale bars indicate 40 µm.

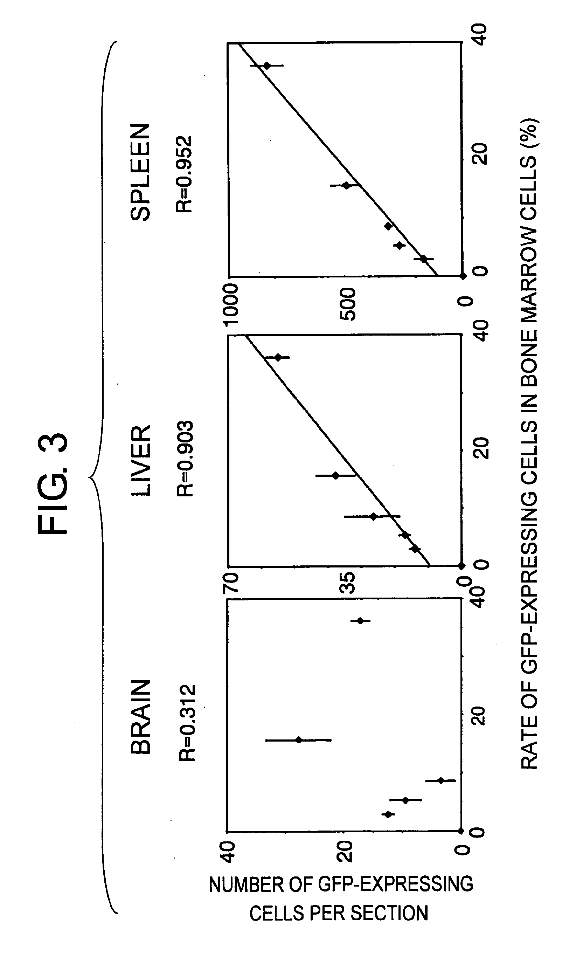

Fig. 3 shows graphs indicating the correlations between GFP-expressing cells in the bone marrow and GFP-expressing cells that translocated to tissues in mice on day 7 after transplantation. Positive correlations were observed in peripheral tissues such as liver and spleen, whereas absolutely no correlation was observed in the brain.

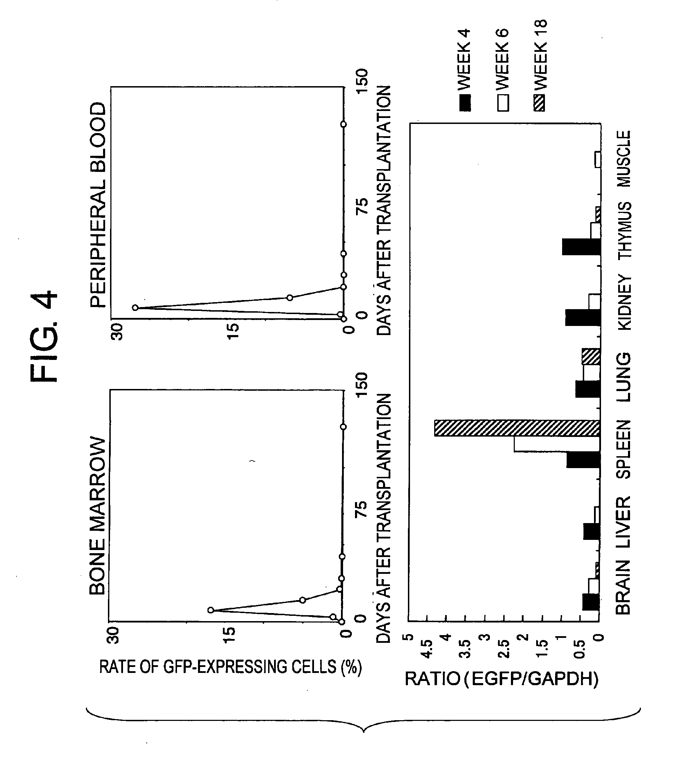

Fig. 4 shows graphs indicating the results of time-course analyses on the tissue distribution of GFP-expressing cells. The upper two graphs show the change over time in the distribution of GFP-expressing cells in bone marrow and peripheral blood. The lower graph shows the changes over time (at weeks 4, 6, and 18) in the distribution of GFP-expressing cells in each tissue (brain, liver, spleen, lung, kidney, thymus, and muscle).

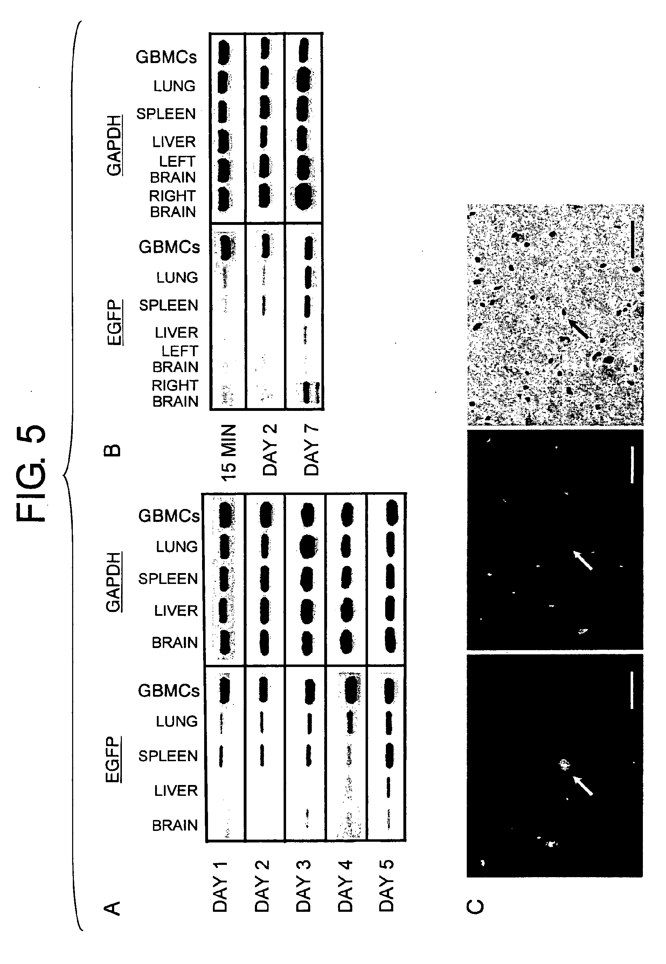

Fig. 5 shows photographs indicating the results of analysis of the time of brain-localization of GFP-expressing cells. (A) is a photograph showing the results of examining GFP distribution in tissues (brain, liver, spleen, lung, and GBMCs) from days 1 to 5 after transplantation. (B) is a photograph showing the result of examining the distribution of GFP-expressing cells (in the left brain, right brain, liver, spleen, lung, and GBMCs) 15 minutes, two days, and seven days after transplanting cells from the carotid artery. (C) shows micrographs of the brain 15 minutes after transplantation through the carotid artery. The arrows indicate GFP-expressing cells; the photograph on the left is the GFP fluorescence image, the center image is ER-MP12-stained, and the right-hand image is hematoxylin-eosin-stained. The scale bars indicate 40 µm.

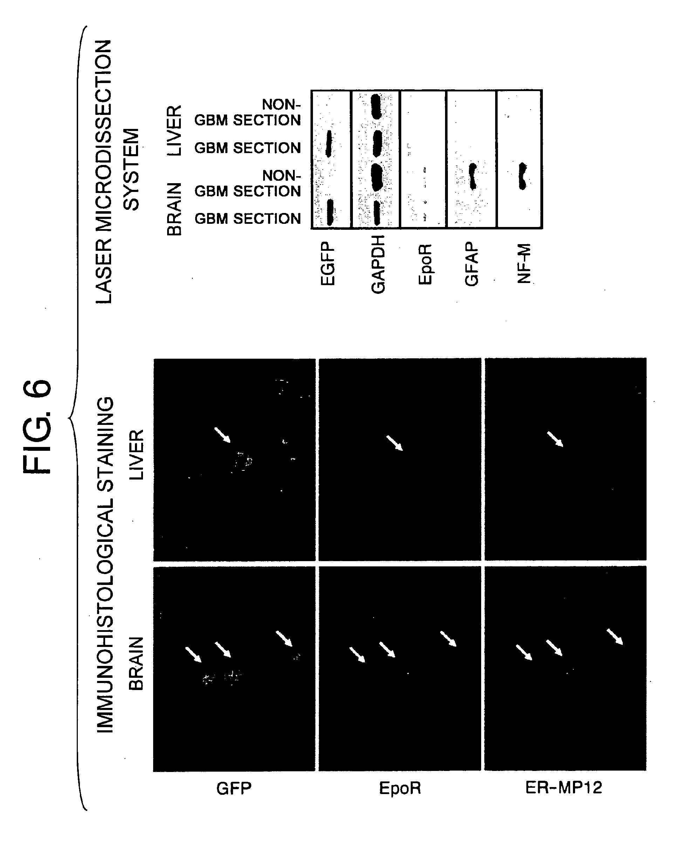

Fig. 6 shows photographs indicating the expression of markers for undifferentiated bone marrow cells in cells that translocated to the brain. Triple staining using antibodies against ER-MP12 and EpoR, which are known as markers for undifferentiated bone marrow cells, was performed on the brain on day 7 after transplantation. A portion of the GFP-expressing cells were GFP-positive, ER-MP12-positive, and EpoR-positive (left). The arrows indicate GFP-expressing cells. The examination of mRNAs in the GFP-expressing cells that translocated to tissues showed that EpoR was expressed in the cells that translocated to the brain (right).

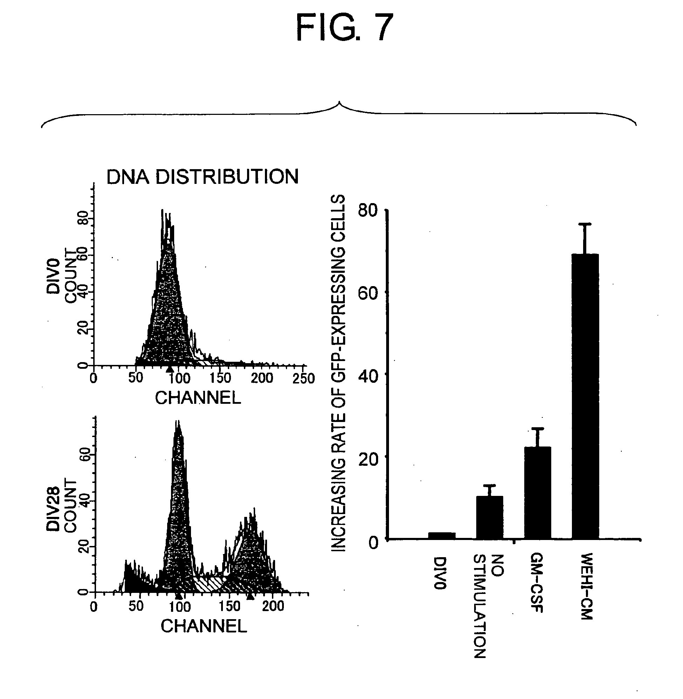

Fig. 7 shows graphs indicating the results of a mixed glial culture that used the entire brain on day 7 after transplantation. On day 7 after transplantation, the brain was treated with enzymes to disperse the cells, and the cells were cultured. The DNA contents of the GFP-expressing cells before and after culturing were examined (left). Culturing for 28 days in a growth factor-supplemented system resulted in a significant increase in GFP-expressing cells (right).

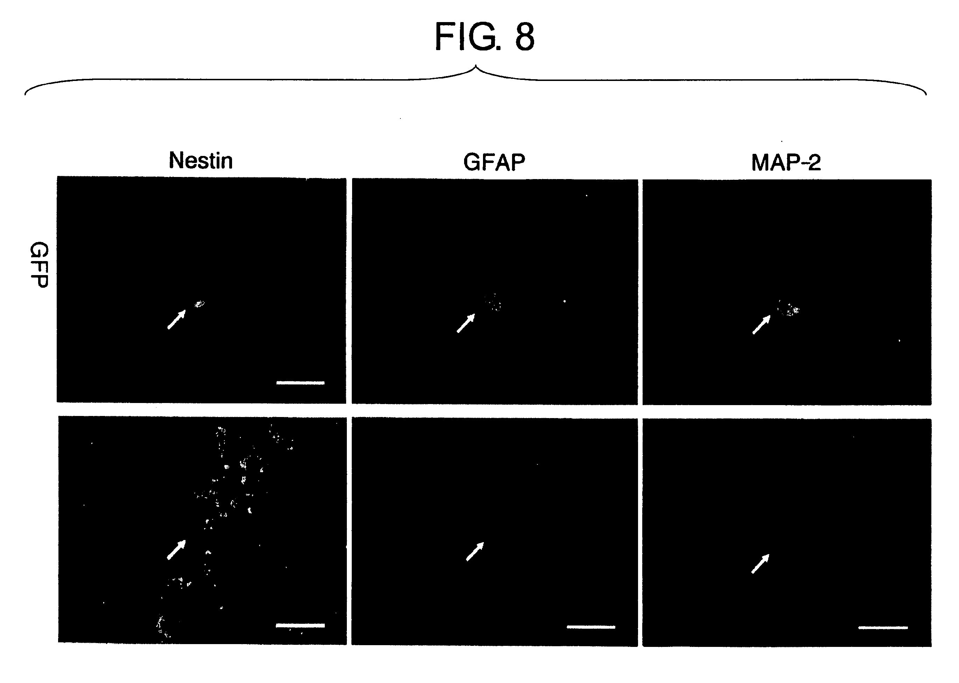

Fig. 8 shows photographs indicating the results of neural cell marker expression in GFP-expressing cells found in the brain on day 7 after transplantation. The expressions of Nestin, a neural stem cell marker; GFAP, an astrocyte marker; and MAP-2, an adult neuron marker, were examined. The arrows indicate identical cells, and the scale bars indicate 40 µm.

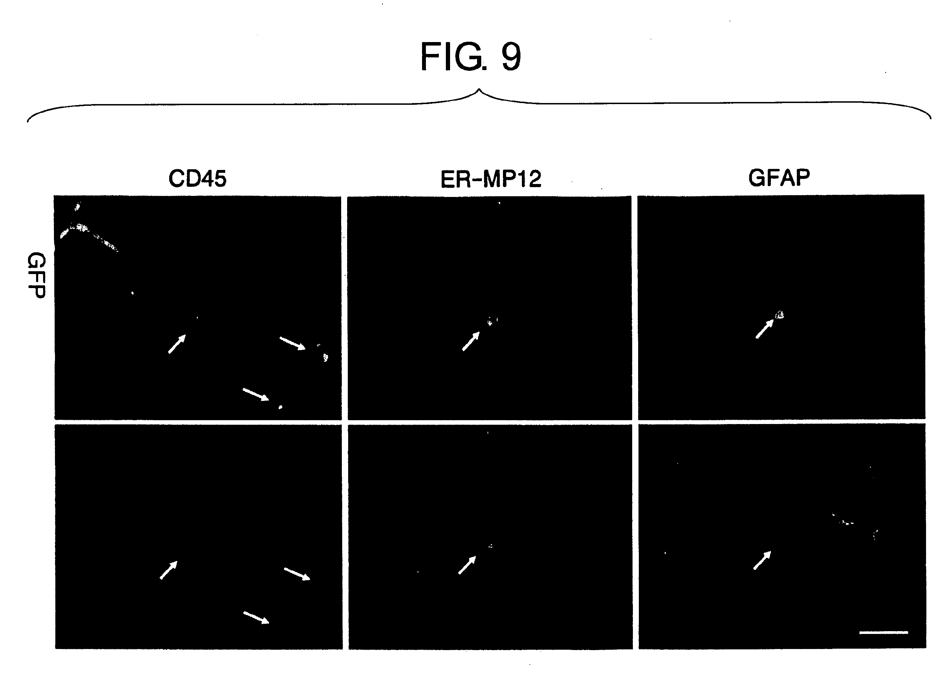

Fig. 9 shows photographs indicating GFP-expressing cells in the brain several months after transplantation. Some of the cells were found to be positive for CD45, a hematopoietic cell marker, or to express undifferentiated cell markers. Cells expressing neural cell markers were not observed.

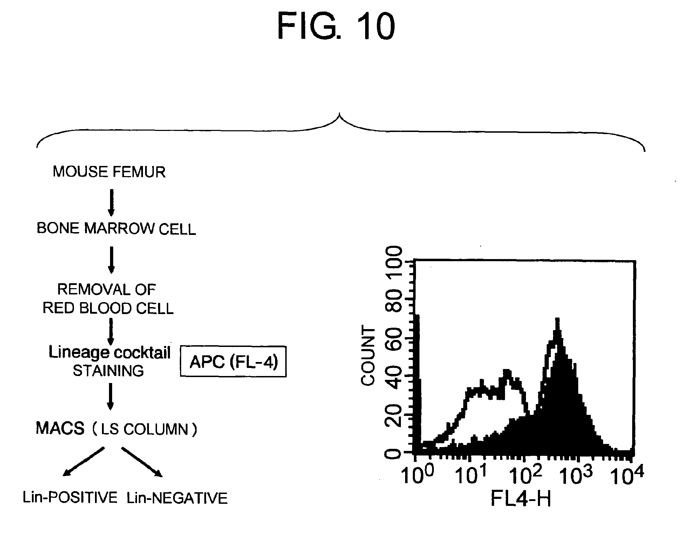

Fig. 10 relates to the separation of lineage-positive/negative fractions.

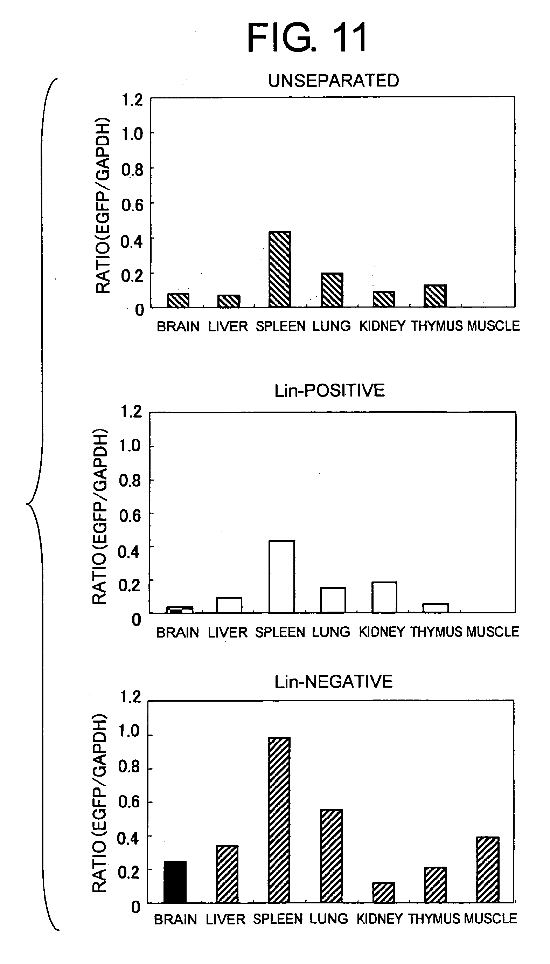

Fig. 11 shows graphs indicating the results of transplanting lineage-positive/negative fractions. Transplantation was performed after separation into lineage-positive fractions and lineage-negative fractions. The tissue distributions of GFP-expressing cells (in brain, liver, spleen, lung, kidney, thymus, and muscle) were examined on day 3 after transplantation. The top, middle, and bottom graphs show the results of transplanting un-fractionated cells, lineage-positive cells, and lineage-negative cells, respectively.

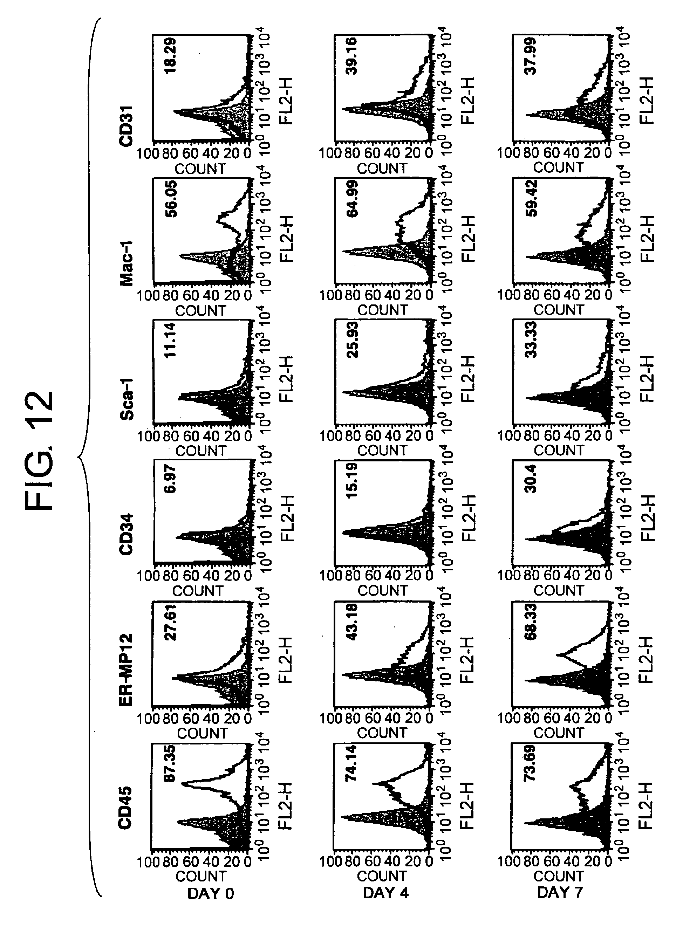

Fig. 12 shows graphs indicating changes in the surface antigen expression of bone marrow cells in a system supplemented with a WEHI-3B cell culture supernatant. Surface antigens on days 0 to 7 after culturing were analyzed by FACS. The numbers indicated in the histograms represent the positive proportions.

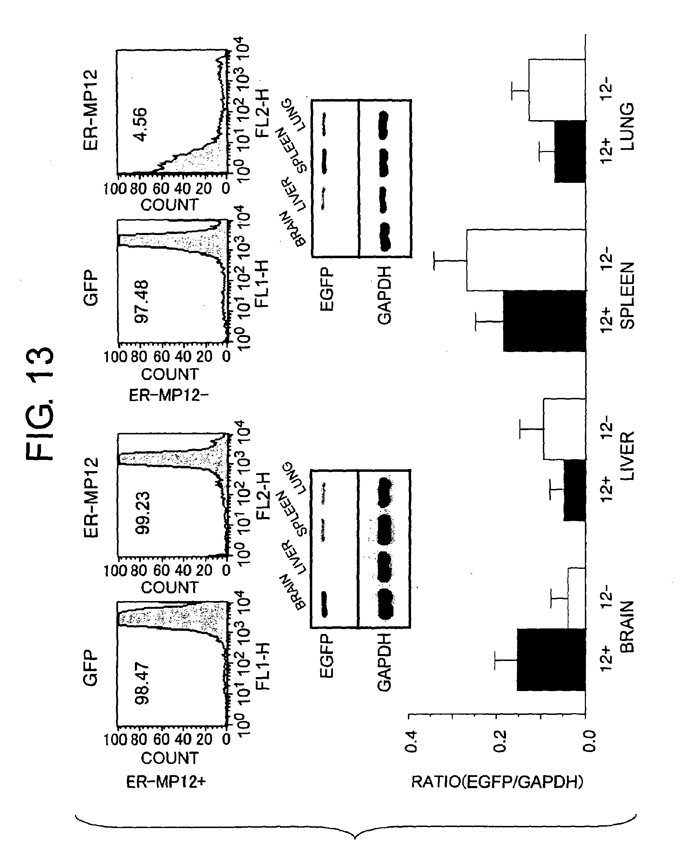

Fig. 13 shows graphs and photographs indicating the results of separating and transplanting ER-MP 12-positive/negative fractions. ER-MP 12-positive fractions and ER-MP12-negative fractions were separated by MACS (top row), and transplantation was performed through the tail vein. In the ER-MP12-positive fractions, the proportion of cells that translocated to the brain increased and the proportion of cells that translocated to the peripheral tissues decreased.

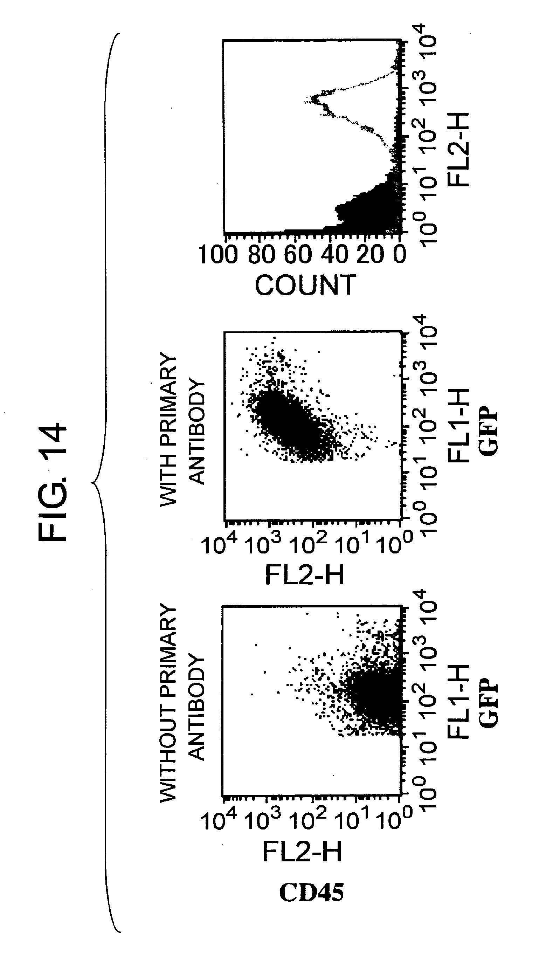

Fig. 14 shows the results of separating Lin-negative fractions from GFP-expressing bone marrow cells, staining for CD45, which is a hematopoietic cell marker, and then analyzing using FACS. The histogram with signal peaks mainly on the left indicates the results in the absence of a primary antibody, and the histogram having signal peaks on the right indicates the result in the presence of a primary antibody. 99% to 100% of the cells are shown to be CD45-positive.

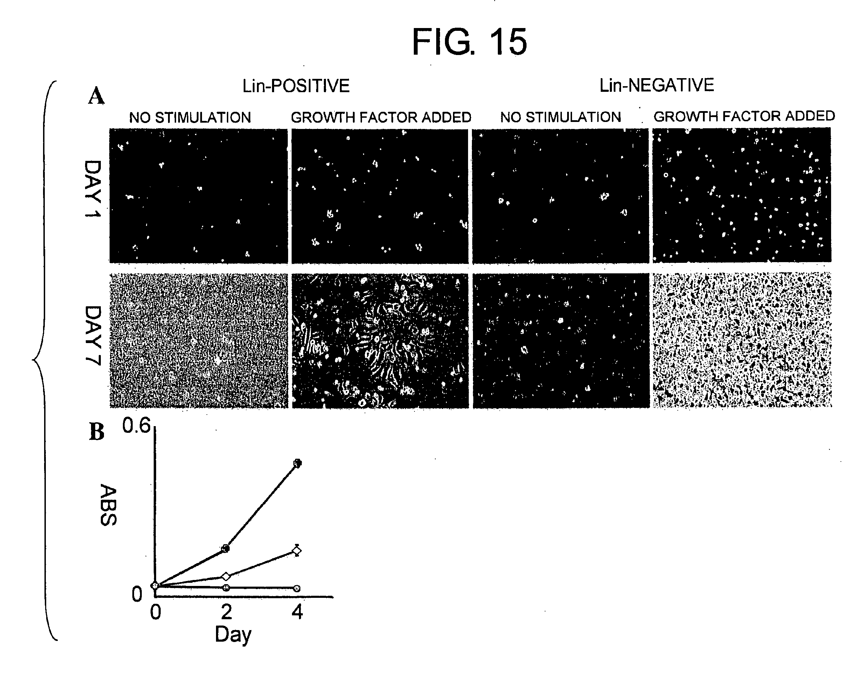

Fig. 15 shows photographs and a diagram indicating the growth of bone marrow cells in the Lin-negative fractions. (A) Lin-negative fractions and Lin-positive fractions were cultured in WEHI-CM (containing a lot of IL-3). (B) Growth of the Lin-negative fractions with WEHI-CM addition (top line) and with rmEPO stimulation (middle line) was examined using a WST assay. The bottom line shows the results for no stimulation.

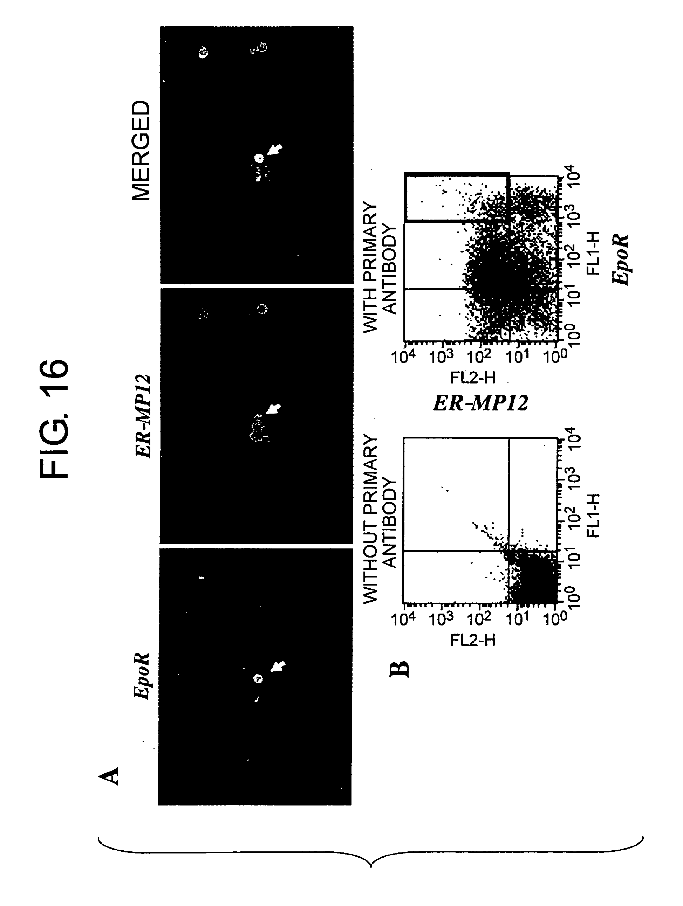

Fig. 16 shows photographs and diagrams indicating (A) fluorescence images and (B) the results of FACS analysis when Lin-negative bone marrow cells were stained with an anti-EpoR antibody and anti-ER-MP12 antibody. Nuclear staining was performed with Hoechst 33342. The magnification is x 400.

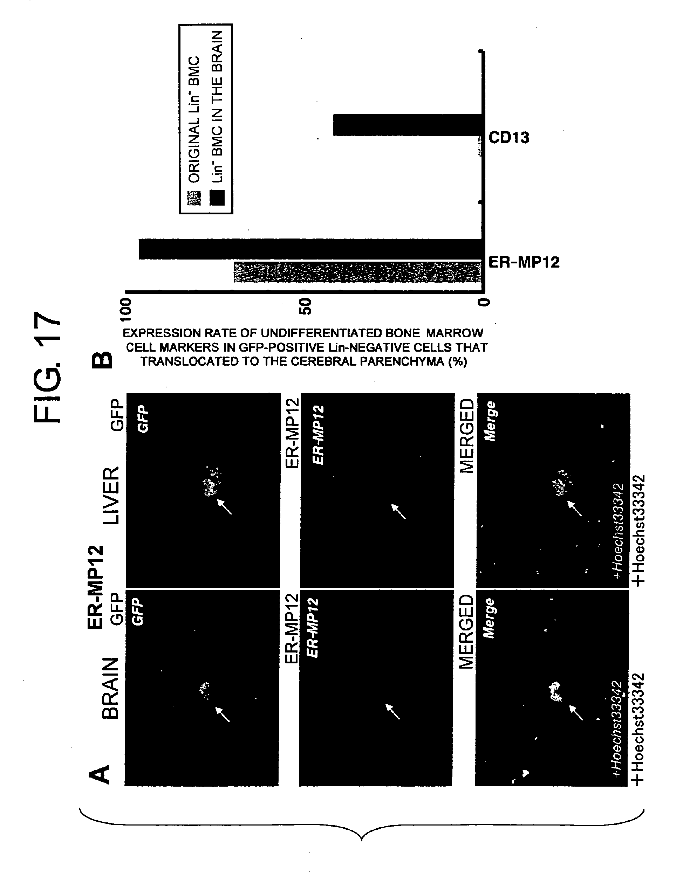

Fig. 17 shows photographs and a diagram indicating the results of analyzing the expression of an undifferentiated bone marrow cell marker in GFP-expressing cells that translocated into the brain when Lin-negative bone marrow cells were transplanted. (A) shows photographs indicating the expression of ER-MP 12 antigen in the brain and liver when GFP-positive Lin-negative bone marrow cells were transplanted. (B) shows a graph indicating the expression rate of an undifferentiated bone marrow cell marker in GFP-expressing cells.

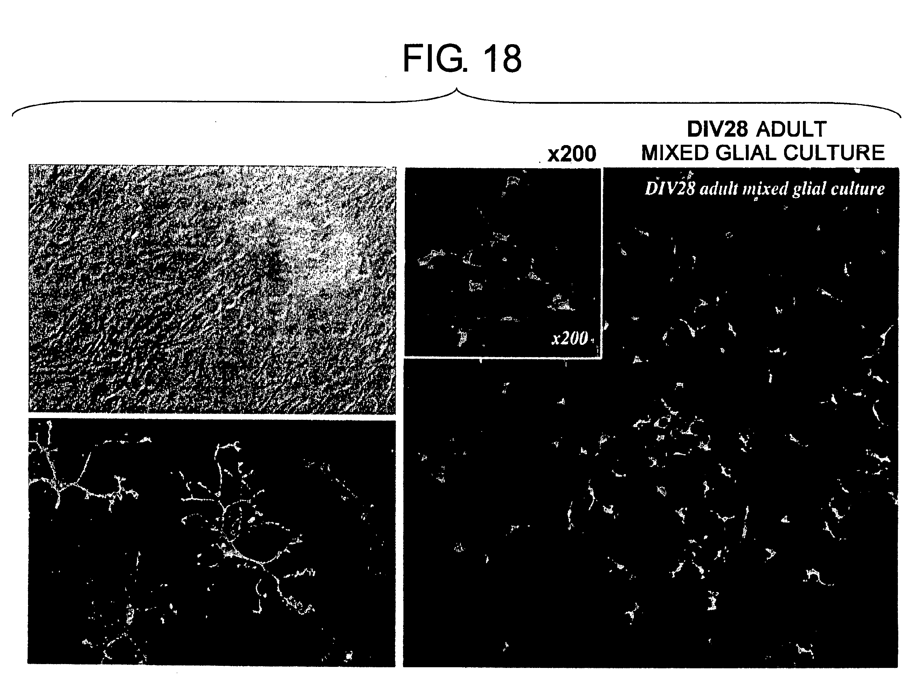

Fig. 18 shows photographs of mixed glial cultures derived from the brain of adult animals. A mixed glial culture derived from the brain of an adult animal was produced, and its differential interference image and fluorescence image after staining with an anti-CD11b antibody (green) and Hoechst 33342 (blue) are shown in the upper left and lower left, respectively. A large number of ramified microglia were confirmed. Furthermore, GFP-expressing bone marrow cells were transplanted intravenously in to an adult animal and a mixed glial culture was produced on day 7 by the same procedure. The fluorescence image of this culture is shown on the right. Many ramified microglia-like bone marrow-derived cells could be confirmed.

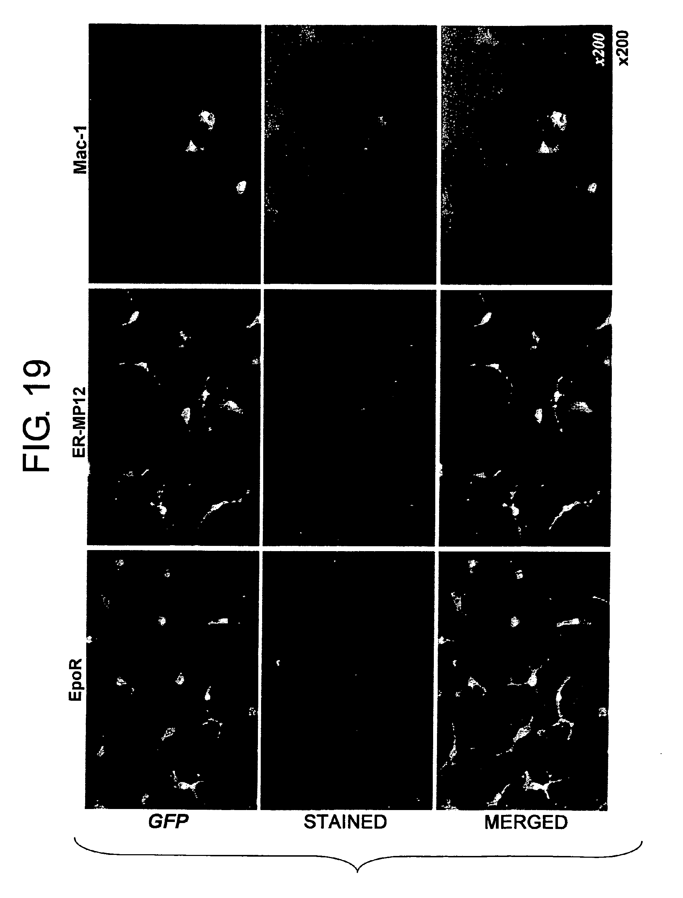

Fig. 19 shows photographs indicating the results of analyzing the expression of antigens in GFP-expressing cells present in a mixed glial culture.

### Best Mode for Carrying Out the Invention

The present inventors discovered that cells comprised in the cell fractions prepared from bone marrow cells have brain-localizing activity.

The present invention provides bone marrow cells with brain-localizing activity, or cells with brain-localizing activity or cell fractions substantially comprising these cells, which are prepared from bone marrow or bone marrow cells.

In the present invention, bone marrow cells (BMCs) refer to cells present in the bone marrow (a group of cells, or a cell population). Bone marrow cells are mainly composed of hematopoietic cells, and mesenchymal cells such as stromal cells that support these cells. In a narrow sense, the phrase "bone marrow progenitor cells" refers to hematopoietic progenitor cells, but in a broad sense it may be considered to comprise mesenchymal progenitor cells. In a preferred embodiment, the cells of the present invention are demonstrated to be positive for CD45, a hematopoietic cell marker, before and after transplantation. Thus, the bone marrow progenitor cells of the present invention do not preferably comprise mesenchymal cells. In addition, the "bone marrow cells" may also be referred to as myeloid cells or myelogenic cells in the present invention.

"Bone marrow" usually refers to tissues that exist in cavities (medullary cavities) formed by osteoclasts in bone tissues, and is a major hematopoietic tissue. Bone marrow is composed of cell components such as blood cells, stromal cells, adipocytes, osteoblasts and osteoclasts, vascular endothelial cells, and nerve tissues, as well as extracellular matrix components such as collagen, proteoglycan, and fibronectin. In other words, "bone marrow cells" in the present invention usually refers to cell components in the bone marrow.

Cells of the present invention can be prepared using both bone marrow and bone marrow cells as a starting material.

An aggregate of cells comprising collagen can be excluded by passage through a nylon mesh before preparing brain-localizing cells. However, matrix components such as collagen that pass through such a mesh do not always have to be removed in advance. For example, bone marrow cells can be prepared from the bone marrow such that the extracellular components in the bone marrow are removed by using a method such as that described below, but the method is not limited thereto:
(1) collecting femurs and cutting off both ends;
(2) inserting a syringe equipped with a needle from one end, and propelling the cells using a buffer;
(3) collecting bone marrow cells in the buffer using centrifugation, and removing erythrocytes using NH₄Cl buffer; and
(4) washing the cells twice with the buffer, and then using the cells that passed through a nylon mesh as bone marrow cells to prepare the brain-localizing cells of the present invention.

The bone marrow cells of the present invention are usually collected from the bone marrow of mammals. The mammals from which the bone marrow cells are collected are not particularly limited, as long as they have bone marrow, and examples include humans, monkeys, mice, and rats, but humans are preferred. In an embodiment of the present invention, the animals (recipients) to be administered with the cells of the present invention, and the animals (donors) whose cells are collected preferably belong to the same species. Furthermore when using cells of the present invention on a human, for example, the cells of the present invention are most preferably cells collected (derived) from the bone marrow of that human subject himself/herself.

The bone marrow cells of the present invention with brain-localizing activity are preferably the cells (bone marrow progenitor cells) comprised in the progenitor cell fraction of bone marrow cells (bone marrow progenitor cell fraction). In the present invention, these cells may be referred to as "bone marrow progenitor cells".

One of the characteristics of the bone marrow progenitor cells of the present invention with brain-localizing activity is that they are undifferentiated cells. Therefore, the present invention provides bone marrow progenitor cells that are undifferentiated and have brain-localizing activity.

Hemocytes in bone marrow are known to terminally differentiate into blood cells such as monocytes or macrophages *via* stem cells and progenitor cells. Progenitor cells have the ability to differentiate into a number of cell populations, and as they progress through the stages of differentiation, they are only destined to become particular cells, such as granulocytes or monocytes. The brain-localizing cells of the present invention are usually negative for a lineage cocktail (Lin-), which is a collection of antibodies against antigens expressed on the aforementioned "particular cells". Therefore, the brain-localizing cells of the present invention are usually cells that have not progressed through the differentiation stages, and are preferably undifferentiated cells. More specifically, in a preferred embodiment of the present invention, differentiated cells are removed using the lineage cocktail, which is a collection of antibodies against antigens expressed on the "particular cells", and a population of undifferentiated cells is separated.

For example, the undifferentiated cells of the present invention have the following characteristics:
(a) ER-MP12-positive (ER-MP12+); and/or
(b) Lin-negative (Lin-)

The undifferentiated cells of the present invention preferably have either one of the above-mentioned characteristics, but more preferably, they have both characteristics (a) and (b).

Furthermore, the present inventors discovered that when Lin-negative fractions and Lin-positive fractions are individually cultured in the presence of growth factors, Lin-negative fractions show very high growth potential. Therefore, in the present invention, Lin-negative cell fractions are preferably cultured with the addition of growth factors, but this is not required. The cells can also be grown using growth factors that act on different receptors.

Cell markers such as Sca-1 and CD133 are usually expressed in undifferentiated cells; therefore, an embodiment of the undifferentiated cells of the present invention may include cells that are Sca-1-positive and/or CD133-positive.

In a preferred embodiment, the brain-localizing cells of the present invention are (c) EpoR-positive. The brain-localizing cells of the present invention are usually cells comprising at least one or more of the characteristics (a) to (c) mentioned above, are preferably cells comprising at least two or more of these characteristics, and are more preferably cells comprising all of the above-mentioned characteristics, (a) to (c).

Furthermore, brain-localizing cells of the present invention are preferably CD13-positive.

In addition, an embodiment of the cells of the present invention is cells expressing CD45. Usually, a cell marker CD45 is not expressed in mesenchymal cells; therefore, an embodiment of the brain-localizing cells of the present invention is bone marrow cells that are undifferentiated cells and are not mesenchymal cells. Thus, in a preferred embodiment of the present invention, the cells are undifferentiated and (d) CD45-positive.

Furthermore, the bone marrow progenitor cells of the present invention have characteristics (functions) such as the following:
(1) when the cells of the present invention are administered to a recipient (host), the cells have the function (characteristic) of translocating to the brain without going *via* the host bone marrow;
(2) after translocating to the brain, the cells have the function (characteristic) of arresting in their G0/G1 phase in the cerebral parenchyma and existing in a growth-arrested state;
(3) the cells have the function (characteristic) of not transdifferentiating into neural cells in the cerebral parenchyma; and
(4) the cells have the ability to pass through the blood brain barrier.

These characteristics were discovered by the present inventors.

In general, the transfer of substances from blood into the brain tissues is restricted by a structure called the blood-brain barrier (BBB). This structure protects the brain from harmful substances and the like. In the present invention, "brain-localizing activity" refers to the activity of cells administered (for example intravenously) into the body of an organism (a mammal) to translocate into brain tissues. The cells of the present invention can usually be described as cells that have brain-localizing activity (brain-localizing cells), but they can also be described, for example, as cells with the ability to pass through the blood-brain barrier.

More specifically, "translocation to the brain" in the present invention refers to translocation to the cerebral parenchyma or brain tissues.

The mechanism for passing through the blood-brain barrier is presumed to be transmigration. Therefore, cells of the present invention may be described, for example, as cells with the ability to induce transmigration (transcytosis), but the cells of the present invention are not to be construed as being limited to cells with such activity.

"Transmigration" refers to a phenomenon in which certain molecules penetrate into the brain by passing through vascular endothelial cells rather than the intercellular spaces of the vascular endothelial cells. This is also called "transendothelial cell migration", the "transcellular pathway", or "transcytosis". The molecules (cells and such) that pass through vascular endothelial cells *via* this mechanism may have signal molecules on their surface, and induce the above-mentioned phenomenon in the vascular endothelial cells *via* receptors on their cell surface.

In the present invention, for example, when cells are obtained from mouse bone marrow, the cells can be determined to have brain-localizing activity by introducing a marker gene (for example, GFP) into the test cells, then administering the cells *via* the tail vein, and then detecting the presence of the marker in the brain.

The present invention also relates to methods for preparing (obtaining or concentrating) brain-localizing cells or cell fractions that substantially comprise these cells from bone marrow or bone marrow cells. More specifically, the present invention provides methods for obtaining brain-localizing cells, where the methods comprise the step of separating bone marrow progenitor cells or bone marrow progenitor cell fractions from bone marrow or bone marrow cells. The present invention also provides brain-localizing cells and cell fractions substantially comprising brain-localizing cells that are obtained by these methods. The cell fractions of the present invention are prepared from bone marrow or bone marrow cells, and are cell fractions (bone marrow progenitor fractions) that substantially comprise the cells of the present invention. Cell components in the cell fractions of the present invention preferably comprise the various characteristics described above.

The present inventors discovered that the brain-localizing cells of the present invention exist in undifferentiated bone marrow progenitor cell fractions in bone marrow cells. Therefore, the methods of the present invention described above comprise the step of preparing undifferentiated cells (fractions) from bone marrow or bone marrow cells.

Undifferentiated cells (fractions) can be prepared, for example, by separation and collection using the characteristics (cell markers and such) of undifferentiated cells. For example, a lineage cocktail, which is a collection of antibodies that recognize antigens against differentiated cells, can be suitably used.

Positivity for ER-MP12, a marker for undifferentiated bone marrow cells, can be used as an indicator to separate and collect undifferentiated cells (fractions) in the above-mentioned methods. For example, an anti-ER-MP 12 antibody can be used to separate ER-MP12-positive cells.

Markers for undifferentiated cells, such as Sca-1 and CD133 can be used to separate undifferentiated cells (fractions). More specifically, a preferred embodiment of the methods of the present invention is methods comprising the step of separating Sca-1-positive cells and/or CD133-positive cells. This step can be performed, for example, by using an anti-Sca-1 antibody or an anti-CD133 antibody in general molecular biological techniques that utilize antigen-antibody reactions.

The methods of the present invention are preferably methods comprising the step of separating EpoR-positive cells or cell fractions substantially comprising such cells, and more preferably methods that further comprise, in addition to the above-mentioned step, the step of separating the above-described undifferentiated cells or cell fractions substantially comprising such cells. EpoR-positive cells can be separated, for example, by using an anti-EpoR antibody.

Specific examples of the methods of the present invention that use the above-described lineage cocktail or various antibodies include the methods described in the following Examples.

As a result of examining conditions that amplify bone marrow-derived ER-MP12-positive cells in cultures, the present inventors discovered that ER-MP12-positive cells are amplified when cultured for a few days in a system supplemented with a WEHI-3B cell culture supernatant.

Therefore, a preferred embodiment of the preparation methods of the present invention is methods further comprising, in addition to the above-mentioned steps, the step of culturing cells with the addition of a WEHI-3B cell culture supernatant. This step is more specifically a step of adding a WEHI-3B cell culture supernatant to ER-MP12-positive cells or to cell fractions substantially comprising such cells, and then culturing the cells for a few days. The aforementioned phrase "for a few days" usually refers to three to 14 days or so, preferably five to ten days, and more preferably six to eight days, and most preferably seven days.

Furthermore, a preferred embodiment of the methods of the present invention is methods further comprising, in addition to the above-mentioned steps, the step of separating CD45-positive cells. This step can be performed, for example, using general molecular biological techniques that utilize antigen-antibody reactions using an anti-CD45 antibody.

Moreover, the methods of the present invention further comprise methods that comprise the step of separating CD13-positive cells, in addition to the above-mentioned steps. This step can be performed, for example, using general molecular biological techniques that utilize antigen-antibody reactions using an anti-CD 13 antibody.

In the methods of the present invention; positive cells (fractions) and negative cells (fractions) can be separated by those skilled in the art using general cell separation techniques, such as MACS and FACS.

When the subject animals are nonhuman animals such as mice, the brain-localizing cells of the present invention can be obtained, for example, as follows:

Whole bone marrow cells or partially purified cells are transplanted into a subject animal, then one week or so later, a cell culture comprising brain-localizing cells is produced from its brain, and foreign brain-localizing cells that translocated to the brain due to the transplantation are cultured in the presence of WEHI-CM or various growth factors that act specifically on bone marrow progenitor cells to prepare concentrated cells (neurons and glial cells, other than the undifferentiated microglia that are originally present in the brain, do not grow under these conditions). The present inventors confirmed that when cells thus obtained are transplanted again, they show brain-localizing properties. In an example of these methods, bone marrow cells derived from EGPF transgenic mice can be used as the bone marrow cells to be transplanted, so that the foreign cells can be identified by the fluorescence of EGPF.

The brain-localizing cells of the present invention can be used to transport a desired substance (compound) to the brain. More specifically, by introducing a desired substance into the brain-localizing cells of the present invention, or by linking it to such cells, the substance can be translocated into the brain.

Thus, the present invention provides carriers for delivery to the brain, which comprise as active ingredients the brain-localizing cells (bone marrow progenitor cells) of the present invention, cell fractions substantially comprising these cells, or cells obtained by the methods of the present invention. These carriers may also be referred to as "supports" or "transporters".

The substances supported by the carriers are not particularly limited, but usually, they are preferably biologically active substances. Biologically active substances are, for example, substances that are active in the brain, and they are preferably substances with therapeutic or preventive effects on brain diseases (such as cranial nerve diseases). Preferred embodiments of these substances include nucleic acids such as DNAs (including vectors) or RNAs (including antisense RNAs and siRNAs). For example, DNAs encoding proteins with therapeutic effects on brain disease are an example of the above-mentioned biologically active substances.

The carriers of the present invention can be used to translocate desired pharmaceutical agents to the brain. For example, by using the above-mentioned carriers to support (contain) compounds (pharmaceutical compositions) that have therapeutic effects on brain diseases, the compounds can be efficiently delivered to the brain and can exert effective therapeutic effects. Carriers supporting such compounds (pharmaceutical compositions) themselves are expected to be therapeutic agents for brain diseases. Accordingly, the present invention provides therapeutic agents for brain diseases that comprise structures in which drugs are supported on carriers of the present invention for delivery to the brain. "Supported" may refer to conditions whereby drugs are directly bound to carriers, or conditions in which drugs (pharmaceutical compositions) are contained within (introduced into) carriers.

Examples of substances to be delivered to the brain by the brain-localizing cells (carriers) of the present invention are basically various substances suitable for brain diseases or disorders. For example, drugs that may be considered include neurotrophic factors such as BDNF or GDNF, inhibitory cytokines such as IL-10 or TGF-β, and drugs such as dopamine for treating Parkinson's disease, or donepezil hydrochloride for treating Alzheimer's disease. As demonstrated by levodopa, a prodrug of dopamine, most drugs cannot translocate to the brain unless they are modified (they are degraded prior to translocation to the brain). Thus, by using the cells of the present invention, it may become possible to use drugs that are effective *in vitro* but do not show their effectiveness *in vivo.* Furthermore, doses of commonly used drugs such as donepezil hydrochloride, a therapeutic agent for Alzheimer-type dementia, can be reduced by direct translocation to the brain.

Therapeutic strategies for treatment of brain diseases using the brain-localizing cells of the present invention can be applied, for example, to the following five cases.
(1) Replacement therapies that supplement enzymes and bioactive proteins whose amounts and activities have decreased due to deletions or mutations
   These therapies are performed for various brain diseases caused by deficiencies of particular enzymes or proteins in the brain due to genetic deletions or mutations, by injecting cells into which genes encoding the deficient proteins and enzymes have been introduced. For degenerative loss of particular neurons in Parkinson's disease and Alzheimer's disease, genes that promote synthesis of neurotransmitters which become deficient due to the degenerative loss of nerves may be used; for example, genes of enzymes involved in dopamine biosynthesis, including tyrosine hydroxylase and biopterin synthase, may be used for Parkinson's disease.
(2) Protective therapies for protecting neurons that would otherwise be lost by degeneration or such, and for strengthening their functions
   These therapies may be performed by injecting cells expressing genes of neurotrophic factors such as NGF, BDNF, GDNF, and NT3 (BDNF and GNNF are known to be particularly effective for Parkinson's disease), which suppress neuron death by various causes including degenerative diseases and cerebral ischemia, and promote the regeneration of neurites. Furthermore, therapies for diseases that involve immune cells, such as multiple sclerosis, may be performed by introducing the TGF-β gene or IL-10 gene, which has immunosuppressive effects, into the cells of the present invention.
(3) Methods for removing tumors, blood clots and such
   These methods may be performed by transferring into the brain the cells of the present invention that express factors having antitumor effects or are introduced with an antitumor agent. Fibrinolytic enzymes may be expressed for the removal of blood clots.
(4) Methods for introducing effective drugs exclusively into the brain
   Some of the drugs that act on the nervous system have high peripheral toxicity, some act on the peripheral nervous system, and others cannot easily pass through the blood-brain barrier; therefore, drug delivery systems specific to the brain are required. Using the cells of the present invention, drugs may be administered specifically to the brain, with little effect on peripheral organs.
(5) Uses as systems for preventing brain disease
   (Such uses are available since bone marrow progenitor cells can be induced to differentiate into microglia-like cells.) Microglias are originally cells that gather at degenerated or inflammatory sites to remove dead cells and are involved in damage repair. They also have antitumor effects and antiviral effects, and can thus be referred to as an intracerebral defense system. Therefore, microglia can be applied not only to the treatment of a single disease, by strengthening these properties through genetic engineering and such, but also to preventive measures against various diseases, by strengthening the intracerebral defense system itself.

Diseases that can be treated using the brain-localizing cells of the present invention are not particularly limited, but examples include Sandhoff's disease and Gaucher's disease. These diseases are also called lysosomal diseases, because in these diseases enzymes and proteins in the lysosome are defective, and substances that should be metabolized accumulate in the lysosome and induce various symptoms. Currently, methods for treating these diseases are mainly enzyme replacement therapies in which enzymes are infused into the bloodstream to decrease the symptoms. Since the brain-localizing cells of the present invention can normally produce enzymes, if these cells can be used against lysosomal diseases, they enable cell therapy without the transport of drugs or genes or such, since they compensate for the role of enzyme-defective cells.

When these cells are used to transport genes or drugs, they may be directed to a wide variety of diseases, such as brain tumors, cerebral ischemia, Parkinson's disease, or Alzheimer's disease. For cerebral ischemia, neuroprotective effects can be locally promoted by erythropoietin, neurotrophic factors including BDNF, and such. In addition, for Parkinson's disease, which is a chronic disease, dopamine can be locally supplemented. Side effects are serious problems in treating brain tumors since drugs do not translocate to the tumor site, and further, most anticancer agents act on proliferative cells (they have no selectivity for cancer); however, these problems can be avoided.

As described above, the present invention provides brain-localizing pharmaceutical agents (brain-localizing pharmaceutical compositions), which comprise biologically active substances comprised in the brain-localizing cells of the present invention (carriers for delivery to the brain). The biologically active substances are usually substances (compounds) with therapeutic or preventive effects against brain diseases.

The pharmaceutical agents of the present invention may be formulated using known pharmaceutical production methods. For example, the agents can be formulated into pharmaceutical formulations suitable for effective administration to living bodies, such as injections, transnasal formulations, transdermal formulations, and oral formulations, but preferably injections, by suitably combining with appropriate commonly used carriers or vehicles, such as sterilized water, physiological saline, vegetable oils (for example, sesame oil and olive oil), coloring agents, emulsifiers (for example, cholesterol), suspending agents (for example gum arabic), surfactants (for example, polyoxyethylene hardened castor oil surfactants), solubilizing agents (for example, sodium phosphate), stabilizers (for example, sugars, sugar alcohols, and albumin), or preservatives (for example, paraben). For example, injections can be provided as freeze-dried products, solutions for injection, or such.

Furthermore, administration into the body can be performed, for example, by intraarterial injections, intravenous injections, or subcutaneous injections, as well as intranasally, transbronchially, intramuscularly, or orally, using methods known to those skilled in the art; however, administration is preferably intra-arterial or intravenous. The doses vary depending on the administration methods, age, weight, and symptoms of the patients and such, and those skilled in the art can appropriately select suitable doses.

In addition, the present invention relates to methods for producing brain-localizing cells comprising biologically active substances. A preferred embodiment of these production methods is methods comprising the step of introducing or binding a biologically active substance to a carrier of the present invention.

More specifically, a preferred embodiment is production methods comprising the steps of (a) obtaining brain-localizing cells from bone marrow or bone marrow cells using a method of the present invention, and (b) introducing a biologically active substance into the brain-localizing cells of (a).

Furthermore, the present invention provides kits for producing the brain-localizing cells of the present invention, and kits for producing the brain-localizing cells that comprise biologically active substances of the present invention.

Kits of the present invention may comprise components such as an anti-ER-MP12 antibody, lineage cocktail, anti-EpoR antibody, anti-Sca-1 antibody, anti-CD133 antibody, anti-CD45 antibody, medium for culturing bone marrow cells, or such.

A preferred embodiment of the kits of the present invention for preparing brain-localizing cells is kits comprising at least two or more of the following (a) to (c) as components :
(a) an anti-ER-MP12 antibody;
(b) a lineage cocktail;
(c) an anti-EpoR antibody; and
(d) a medium for culturing bone marrow cells.

Furthermore, the kits of the present invention may comprise brain-localizing cells (fractions) of the present invention as samples. The kits may further comprise (e) an anti-CD13 antibody.

The kits of the present invention for producing brain-localizing cells comprising biologically active substances comprise components such as brain-localizing cells (fractions) of the present invention and media for culturing the cells, or reagents for introducing biologically active substances into the cells, or such. Examples of the reagents include, for example, various cell transfection reagents.

A preferred embodiment of the kits of the present invention for producing brain-localizing cells comprising biologically active substances is, for example, kits that comprise at least (a) the components described below, and more preferably at least the following components (a) and (b):
(a) the brain-localizing cells (bone marrow progenitor cells) of the present invention, or cell fractions of the present invention substantially comprising these cells; and
(b) a medium for culturing the brain-localizing cells (bone marrow progenitor cells) of the present invention.

In addition to the above-mentioned components, biologically active substances that are to be introduced into the cells of the present invention can also be packaged into the kits of the present invention.

Various antibodies used in the present invention can be appropriately produced by those skilled in the art using general antibody production techniques. The above-mentioned "lineage cocktail" can also be easily obtained by those skilled in the art. Media commonly used to culture bone marrow cells can be used as the above-mentioned "medium". Those skilled in the art can easily find out the basic composition and such of the "media" from literature, manuals, and such.

Various reagents used for various methods for separating the cells of the present invention, such as MACS and FACS, can be comprised in the kits of the present invention. Specifications of the kits of the present invention, instructions for the methods and such can also be suitably packaged into the kits.

The bone marrow progenitor cells of the present invention can be distinguished by methods for producing these cells. More specifically, a preferred embodiment of the present invention relates to bone marrow progenitor cells with brain-localizing activity, which are prepared by methods comprising the steps of:
(a) separating bone marrow progenitor cells or bone marrow progenitor cell fractions from bone marrow or bone marrow cells;
(b) separating undifferentiated cells, or cell fractions substantially comprising these cells;
(c) separating EpoR-positive and/or CD13-positive cells, or cell fractions substantially comprising these cells; and
(d) culturing these cells with the addition of a WEHI-3B cell culture supernatant.

The present invention also provides compositions that comprise pharmaceutically acceptable carriers in addition to the bone marrow progenitor cells of the present invention, carriers of the present invention for transfer to the brain, or brain-localizing pharmaceutical agents of the present invention.

The present invention further relates to methods for treating brain diseases, which comprise the step of administering individuals (such as patients) with the bone marrow progenitor cells of the present invention, carriers of the present invention for transfer to the brain, or brain-localizing pharmaceutical agents of the present invention.

Generally, administration to an individual can be performed by methods known to those skilled in the art, such as intra-arterial injection, intravenous injection, and subcutaneous injection. The doses vary depending on the administration methods, weight and age of the patients, and such, but those skilled in the art (such as physicians, veterinarians, and pharmacists) can appropriately select suitable doses.

The present invention also relates to uses of the bone marrow progenitor cells of the present invention or carriers of the present invention for transfer into the brain to produce brain-localizing pharmaceutical agents (therapeutic agents for brain diseases).

All prior art documents cited herein are incorporated herein by reference.

### Examples

Herein below, the present invention will be specifically described with reference to Examples, but it is not to be construed as being limited thereto.

### [Example 1] Effects of drug treatment on host blood brain barrier

When bone marrow transplantations are performed, in many cases, host bone marrow cells are removed by radiation exposure. This method is suggested to partially disrupt the blood brain barrier or cause neurological deficits. To perform a bone marrow transplantation that was less invasive to the brain, host bone marrow cells were removed using a drug, 5-FU.

To confirm that the blood brain barrier was undamaged, Evans blue was injected into the tail vein of 5-FU-treated B6 mice, normal B6 mice, and B6 mice that were physically injured using an injection needle, and dye leakage through the blood brain barrier was examined (Fig. 1). As a result, dye leakage was not observed in 5-FU-treated mice, and damage to the blood brain barrier could not be confirmed.

### [Example 2] Correlation between foreign cells found in the cerebral parenchyma and those in the bone marrow on day 7 after transplantation

Bone marrow cells collected from GFP transgenic mice were transplanted via the tail vein of 5-FU-treated mice. GFP-expressing cells were confirmed in the brain, liver, spleen, and lung on day 7 after transplantation (Fig. 2). When correlations between GFP-expressing cells that translocated to the bone marrow and those that translocated to tissues were examined, a positive correlation was observed in peripheral tissues such as the liver or spleen, whereas no correlation was observed in the brain (Fig. 3).

### [Example 3] Examination of the stage of brain-localization of cells

The distributions of GFP-expressing cells in the bone marrow, bloodstream, and tissues were examined over time (Fig. 4). In the bone marrow and blood, the proportion of GFP-expressing cells significantly increased on day 7 after transplantation, and GFP expression had completely disappeared in weeks 3 to 4. GFP-expressing cells that translocated to the liver were virtually undetectable in week 6 after transplantation, and expression had completely disappeared in week 18. In contrast, the GFP-expressing cells that translocated to the brain decreased in number after supply from the bloodstream was terminated; however, expression was still observed in week 18.

To investigate at what stage GFP-expressing cells translocate into the brain, the brains of transplanted mice were collected over time, and GFP expression was examined using RT-PCR methods (Fig. 5). GFP expression was observed in the brain on days 1 to 2 after transplantation. In bone marrow transplantation *via* the tail vein, the bone marrow cells placed into the bloodstream circulate and spread throughout the body before reaching the brain, and thus GFP expression in the brain may have been delayed.

Cells were then injected from one of the carotid arteries so they would circulate first through the brain. Tissue distribution of GFP-expressing cells in this system, where cerebral circulation is followed by systemic circulation, was analyzed over time using RT-PCR methods and microscopic examination of frozen tissue sections. Translocation of cells to the brain was found 15 minutes after transplantation, and GFP expression was still observed two and seven days later. GFP expression levels showed left-right asymmetry. This appeared to be because the cells were injected through one of the carotid arteries, and thus the hemisphere subjected to first circulation showed stronger expression.

### [Example 4] Analysis on the properties of brain-localizing cells

To examine the differences between GFP-expressing cells found in the brain and cells that translocate to peripheral tissues on day 7 after transplantation, immunohistochemical analysis was performed on tissue sections prepared after transplantation (Fig. 6). A comparison of GFP-expressing cells that translocated to the brain and those that translocated to the liver showed that the cells in the brain were positive for ER-MP12, a marker of undifferentiated bone marrow cells, but that this marker was not expressed in the liver. When the expression of erythropoietin receptors found in common myeloid progenitor cells or erythrocyte/polynuclear leukocyte progenitor cells was examined, the expression was observed in a portion of GFP-expressing cells in the brain but not in the liver. A similar result was obtained for mRNA expression. When the DNA contents of the cells that translocated to the brain were examined, most cells were shown to be arrested in the G0/G1 phase and to have stopped growing (Fig. 7). After transplantation, the whole brains were subjected to enzyme treatment to disperse the cells, and when the cells were cultured in a 10% serum-supplemented system, the number of GFP-expressing cells increased approximately eight times. The increase in the number of cells was approximately 20 times when the system was supplemented with GM-CSF, and approximately 70 times when the system was supplemented with 20% WEHI-3B cell-derived culture supernatant (which is a source of IL-3).

To examine whether GFP-expressing cells that translocate to the brain transdifferentiate into neural cells, tissue sections from seven days to 18 weeks after transplantation were stained with antibodies against Nestin, a marker of neural stem cells; GFAP, a marker of astrocytes; and MAP-2, a marker of mature neurons (Figs. 8 and 9, Table 1). Expression of these markers was observed in endogenous cells, but not in GFP-expressing cells.

**Table 1**

| | Hematopoietic cell markers | | | | Neuronal markers | | |
|---|---|---|---|---|---|---|---|
| | CD45 | CD34 | ER-MP12 | Mac-1 | Nestin | GFAP | MAP-2 |
| Proportion | 27/27 | 0/25 | 36/37 | 39/42 | 0/32 | 0/32 | 0/25 |

In the Table, the "proportion" refers to the total number of stained cells to the number of GFP-expressing cells.

Lin-negative fractions were separated from GFP-expressing bone marrow cells, stained for CD45, a hematopoietic marker, and the cells were analyzed using FACS (Fig. 14). The results showed that the cells of the present invention are CD45-positive.

### [Example 5] Concentration of brain-localizing cells

Since it was assumed that brain-localizing cells are present in undifferentiated bone marrow progenitor cell fractions, the fractions were separated into positive (Lin+) fractions and negative (Lin-) fractions using a lineage cocktail, which is a collection of antibodies that recognize antigens on differentiated cells, and the cells were transplanted (Figs. 10 and 11). As shown in Fig. 11, the brain-localizing activity of Lin-negative fractions was significantly greater than that for the Lin-positive fractions. The negative fractions accounted for approximately 5% of all bone marrow cells. When the negative fractions were stained for markers of undifferentiated bone marrow cells or stem cells, approximately 85% were found to be ER-MP12-positive cells. When Lin-negative cells were transplanted, the proportion of GFP-expressing cells that translocated to the brain increased approximately four times as compared to when un-separated bone marrow cells were transplanted. Separation of Lin-negative cell fractions was shown to be very effective for preparing brain-localizing cells.

GFP-expressing cells that translocated to the brain expressed ER-MP12 antigen; therefore, conditions for amplifying bone marrow-derived ER-MP12-positive cells in culture were examined. When the cells were cultured for seven days in a system supplemented with a WEHI-3B cell culture supernatant, approximately 70% of the cells were shown to be ER-MP12-positive (Figs. 12 and 15). Addition of a growth factor to the Lin-negative fractions increased their productivity.

When transplantation was performed after separation into ER-MP12-positive and ER-MP12-negative fractions using MACS, the proportion of cells that translocated to the brain increased approximately three times as compared to when un-separated bone marrow cells were transplanted (Fig. 13). The proportion of cells that translocated to peripheral tissues was reduced as compared to when un-separated bone marrow cells were transplanted. Meanwhile, when the ER-MP12-negative fractions were transplanted, the proportion of cells that translocated to peripheral tissues increased. As shown in Fig. 13, compared to the ER-MP12-negative cell fractions, ER-MP12-positive cell fractions were shown to have significantly higher brain-localizing activity. Separation of ER-MP12-positive cell fractions was demonstrated to be very effective for preparing brain-localizing cells.

### [Example 6] Identification of brain-localizing cells

A combination of the above-mentioned methods enabled identification of progenitor cells with brain-localizing properties from a bone marrow cell population (Fig. 16A (three-colored fluorescence micrograph) and Fig. 16B (fractionation by FACS histograms)).

An example of the methods of the present invention is as follows.

First, bone marrow cells were collected from mouse femurs and the contaminating red blood cells were removed using red blood cell lysis buffer. Next, a Lineage Cell Depletion Kit was used to stain differentiated cells, and MACS (Magnetic Activated Cell Sorter) was used to collect undifferentiated cell fractions. Separation was confirmed by FACS (Fluorescent Activated Cell Sorter). Next, an anti-ER-MP12 antibody and R-PE-labeled anti-rat IgG antibody were used for staining, and anti-R-PE magnetic beads were used for labeling. Then, Lin-negative ER-MP12-positive fractions were separated using MACS. Separation was confirmed by FACS (Fluorescent Activated Cell Sorter). Next, the cells were reacted with biotinylated erythropoietin or a biotinylated anti-EpoR antibody, and then with anti-biotin magnetic beads. Then, Lin-negative ER-MP12-positive EpoR-positive fractions were separated by MACS. This was followed by reaction with fluorescently-labeled avidin, and separation was confirmed by FACS. The fluorescence images (Fig. 16A) and results of FACS analysis (Fig. 16B) after staining Lin-negative bone marrow cells with an anti-EpoR antibody and anti-ER-MP12 antibody are shown.

### [Example 7] Expression of an undifferentiated bone marrow cell marker in GFP-expressing cells that translocated into the brain when Lin-negative bone marrow cells were transplanted

The expression of an undifferentiated bone marrow cell marker was analyzed in GFP-expressing cells that translocated into the brain when Lin-negative bone marrow cells were transplanted.

The results are shown in Fig. 17. Of the cells derived from the Lin-negative fractions, those cells that entered the brain increased their ER-MP12-positive rate to nearly 100%, and CD13-positive cells, which accounted for only 1% or so of these fractions, were found to increase significantly.

These results suggested that brain-localizing cells can be concentrated by fractionating CD13-positive cells, which account for approximately 1.5% of the Lin-negative fractions. Therefore, when fractionating and isolating the brain-localizing bone marrow progenitor cells of the present invention, the cells of the present invention can be highly concentrated by using the characteristic of being CD13-positive in addition to the above-mentioned characteristics (for example, ER-MP12-positive, Lin-negative, and Epo-positive).

### [Example 8] Mixed glial culture

A mixed glial culture derived from the brain of an adult animal was produced and stained with an anti-CD11b antibody (green) and Hoechst 33342 (blue), and the results are shown in Fig. 18 as a differential interference image (upper left) and a fluorescence image (lower left). A large number of ramified microglia were confirmed. GFP-expressing bone marrow cells were transplanted intravenously to an adult animal and a mixed glial culture was produced on day 7 by the same method. The fluorescence image of this culture is shown in Fig. 18 (right). Many ramified microglia-like bone marrow-derived cells were confirmed.

These results showed that when a mixed glial culture was performed by removing the brain from an animal subjected to bone marrow transplantation, morphologically microglia-like cells such as those shown in Fig. 18 on the left can be collected. More specifically, it was found that those GFP-positive bone marrow cells that transferred into the brain may acquire properties similar to those of microglia.

The expression of antigens in GFP-expressing cells present in a mixed glial culture was analyzed. The results are shown in Fig. 19.

Then, the expression of markers in GFP-expressing cells found in mixed glial cultures was analyzed. The results are shown in Table 2.

**Table 2**

| | EpoR | ER-MP12 | Mac-1 |
|---|---|---|---|
| Untreated | *41*/*197 (20.8%)* | *9*/*161 (5.59%)* | *154*/*170 (90.6%)* |
| +GM-CSF | *101*/*280 (36.1 %)* | *51*/*275 (18.5%)* | *263*/*285 (92.3%)* |
| +WEHI-CM | *308*/*1168 (26.4%)* | *109*/*1053 (10.4%)* | *1335*/*1454 (91.8%)* |

The Table shows the proportions of cells that express the respective markers in GFP-expressing cells. A high percentage of the GFP-expressing bone marrow cells found in a mixed glial culture produced by adding GM-CSF or WEHI-CM were EpoR-positive or ER-MP12-positive.

The above results showed that when bone marrow cells that translocated to the brain were collected and mixed glial cultures were produced, the expression of EPO receptor, ER-MP12, and Mac1 was approximately 20.8%, 5.6%, and 90.6%, respectively; however, expression of these first two increased when the culture was treated with GM-CSF.

### Industrial Applicability

If drugs and such can be effectively transported into the brain using the progenitor cell-derived brain-localizing cells of the present invention, drugs that have been difficult to use since they are metabolized may become usable, or drugs that have to be used at high concentrations may be used at reduced doses. Bone marrow transplantation is an established operation in transplantation therapy, and can be performed relatively easily by intravenous injection or such, and autologous bone marrow cells may also be used to treat various diseases of the brain.

Microglia are known to show brain-specific localization, but since microglia are cells in the cerebral parenchyma, to date, the only way to obtain these cells has been to culture the brain. In contrast, the brain-localizing cells obtained in the present invention can be obtained from bone marrow, from which it is relatively easy to collect cells. Since bone marrow cells can be grown in culture, they can be concentrated *ex vivo* and then transplanted. As is the case for blood transfusions, by producing a stock of bone marrow stem cells, when a disease develops, personalized treatments may become possible, by producing the required number of brain-localizing cells, modifying the cells with a drug or a gene, and then returning them intravenously to the body.

## Claims

1. A bone marrow progenitor cell with brain-localizing activity, wherein the cell is an undifferentiated cell.

2. The cell of claim 1, which is (a) ER-MP12-positive and/or (b) Lin-negative.

3. The cell of claim 2, which is also (c) EpoR-positive.

4. The cell of any of claims 1 to 3, which translocates to the brain without going through a recipient's bone marrow when administered to the recipient.

5. The cell of any of claim 1 to 3, which exists in a growth-arrested state at G0/G1 phase in the cerebral parenchyma.

6. The cell of any of claims 1 to 3, which does not transdifferentiate into a neural cell in the cerebral parenchyma.

7. The cell of any of claims 1 to 3, which has the ability to pass through the blood brain barrier.

8. A bone marrow progenitor cell fraction that substantially comprises cells with brain-localizing activity, wherein the fraction is prepared from bone marrow or bone marrow cells.

9. The cell fraction of claim 8, wherein the cell component is a cell that is ER-MP12-positive and/or Lin-negative.

10. The cell fraction of claim 9, wherein the cell component is a cell that is also EpoR-positive.

11. The cell fraction of any of claims 8 to 10, wherein the cell component is a cell that translocates to the brain without going through a recipient's bone marrow when the cell is administered to the recipient.

12. The cell fraction of any of claims 8 to 10, wherein the cell component is a cell that exists in a growth-arrested state at G0/G1 phase in the cerebral parenchyma.

13. The cell fraction of any of claims 8 to 10, wherein the cell component is a cell that does not transdifferentiate into a neural cell in the cerebral parenchyma.

14. The cell fraction of any of claims 8 to 10, wherein the cell component is a cell with the ability to pass through the blood brain barrier.

15. A method for obtaining a brain-localizing cell, which comprises the step of separating a bone marrow progenitor cell or a bone marrow progenitor cell fraction from bone marrow or bone marrow cells.

16. The method of claim 15, which comprises the step of separating an undifferentiated cell or a cell fraction that substantially comprises said cell.

17. The method of claim 16, wherein the undifferentiated cell is ER-MP12-positive and/or Lin-negative.

18. The method of claim 17, wherein an ER-MP12-positive cell is separated using an anti-ER-MP 12 antibody, and/or an Lin-negative cell is separated using a lineage cocktail.

19. The method of any of claims 16 to 18, which further comprises the step of separating an EpoR-positive cell, or a cell fraction substantially comprising said cell.

20. The method of claim 19, wherein an EpoR-positive cell is separated using an anti-EpoR antibody.

21. The method of any of claims 15 to 20, which further comprises the step of culturing cells with the addition of a WEHI-3B cell culture supernatant.

22. A carrier for delivery to the brain, which comprises the bone marrow progenitor cell of any of claims 1 to 7, the cell fraction of any of claims 8 to 14, or the brain-localizing cell obtained by any of the methods of claims 15 to 21.

23. A brain-localizing pharmaceutical agent, which comprises a biologically active substance that is comprised in the carrier of claim 22 for delivery into the brain.

24. The agent of claim 23, wherein the biologically active substance has a therapeutic effect on a brain disease.

25. A method for producing a brain-localizing cell comprising a biologically active substance, wherein the method comprises the steps of:
(a) obtaining a brain-localizing cell from the bone marrow or bone marrow cells by the method of any of claims 15 to 21; and
(b) introducing a biologically active substance into the brain-localizing cell of step (a).

26. The production method of claim 25, wherein the biologically active substance has a therapeutic effect on a brain disease.

27. The production method of claim 25 or 26, wherein the biologically active substance is a nucleic acid.

28. A kit for preparing a brain-localizing cell, which comprises at least two or more components selected from the following (a) to (d):
(a) an anti-ER-MP12 antibody;
(b) a Lineage cocktail;
(c) an anti-EpoR antibody; and
(d) a medium for culturing a bone marrow cell.

29. A kit for producing a brain-localizing cell, which comprises a biologically active substance, and the following (a) and (b) as components:
(a) a bone marrow progenitor cell of any one of claims 1 to 7, or a cell fraction of any one of claims 8 to 14; and
(b) a medium for culturing a bone marrow progenitor cell of any one of claims 1 to 7.
